# EUROPEAN PATENT APPLICATION

(11) **EP 1 034 803 A1**
(43) Date of publication of application: **13.09.2000**
(21) Application number: 99103929.8
(22) Date of filing: 05.03.1999
(51) Int. Cl.: A61L 15/46

(54) **Breathable absorbent articles having an oxidising agent based odour control system**

(71) Applicant: THE PROCTER & GAMBLE COMPANY, Cincinnati, Ohio 45202 (US)
(72) Inventor: Carlucci, Giovanni, 66100 Chieti (IT); Di Cintio, Achille, 65126 Pescara (IT); Pesce, Antonella, 65120 Pescara (IT)
(74) Representative: Kremer, Véronique Marie Joséphine

(57) **Abstract**

The present invention relates to breathable absorbent articles, such as sanitary napkins and baby diapers having a breathable backsheet, and comprising an odour control system comprising an oxidizing agent. The combination of breathability and the oxidizing agent-containing odour control system delivers an improved odour control performance.

## Description

### Field of the Invention

The present invention relates to absorbent articles, particularly sanitary napkins and panty liners which have an improved odour control system.

### Background of the Invention

Whilst the primary focus of absorbent articles, in particular sanitary napkins remains the ability of these articles to absorb and retain fluids, another important area of development in this field is the control of odorous compounds contained within the absorbed articles during their use. Malodorous compounds typically present in absorbent articles originate from a number of sources. Firstly, the actual components of the bodily fluid discharge such as urine, perspiration, menstrual fluids, menstrual blood and vaginal discharges may themselves contain malodorous compounds. Secondly, malodorous compounds are also generated as a result of the degradation of the components of the bodily fluid discharge contained within the absorbent articles. Consequently, there are a wide range of compounds which may be present at some time during the use of an absorbent article which have an associated malodour. These compounds include volatile fatty acids, ammonia, amines, sulphur containing compounds, alcohols, ketones and aldehydes and numerous derivatives thereof.

The presence and detection of malodorous compounds from absorbent articles during their use, particularly those associated with menstruation may cause the wearer of these products embarrassment. Thus, the prevention of the detection of malodour from such products is highly desirable.

As a result there are numerous disclosures in the art which describe various agents which provide odour control for use in absorbent articles. These odour control agents typically function by physical absorption of the odorous compounds or by chemical interaction with the odorous compounds or precursors of odorous compounds or by masking the odour.

Most of the focus in the prior art is found on the odour absorption technology. Examples of these types of compounds include activated carbons, clays, zeolites, silicates, starches, cyclodextrine, ion exchange resins and various mixture thereof as for example described in EP-A-348 978, EP-A-510 619, WO 91/12029, WO 91/11977, WO89/02698, and/or WO 91/12030. All of these types of odour absorbing agents are believed to control odour by mechanisms whereby the malodorous compounds and their precursors are physically absorbed by the agents and thus such agents hinder the exit of the odour from articles like absorbent articles. However, such mechanisms are not completely effective as the formation of the odour itself is not prevented and thus odour detection is not completely avoided.

However, all of the above described odour control agents have associated drawbacks. Many odour control agents do not provide effective odour control over a broad range of odours. Alternatively, the more effective odour control agents are expensive or such as for example with activated carbon are aesthetically unappealing to the consumer. Also many odour control agents have problems related to their effective incorporation within the absorbent articles.

Hence, there still exists a need to provide alternative odour control agents or systems for effective utilization in absorbent articles. In particular, there exists a need to provide an odour control agent or system which addresses the problem of malodour within absorbent articles when they come into contact with bodily fluids by reducing or even preventing the formation of the malodour.

It has now surprisingly been found that the combination of a breathable absorbent article, particularly by the provision of a breathable backsheet together with an odour control system comprising an oxidising agent provides an unexpected improvement in the odour control performance of the oxidising agent-containing odour control system.

The present invention is based on the finding that the breathability of the absorbent article improves the chemical stability of the oxidising agent. In other words, it has now been observed that the breathability of the absorbent article keeps/retains the oxidizing capacity of the oxidising agent up to the time when the article comes into contact with the bodily fluids. Indeed, the breathability of the absorbent article reduces the hot, humid and occlusive environment between the skin of the wearer and the surface of the absorbent article. This contributes to reduce or even prevent the chemical degradation of the oxidizing agent, that would otherwise have deactivated it. Accordingly, in the present invention, the effectiveness of the oxidizing odour control agent is significantly increased. In other words, the full capacity of the oxidizing odour control agent can be utilized. Hence less oxidising agent may be required within the absorbent article whilst maintaining the required level of odour control.

Furthermore it is believed that other factors contribute to the one mentioned above to deliver the improved odour control benefit associated to the combination of breathability and a selected odour control agent, i.e., oxidising agent.

The combination herein allows to combine different mechanisms resulting in synergistic odour prevention benefit. Indeed, the reduction of the hot, humid and occlusive environment between the skin of the wearer and the surface of the absorbent article due to the breathability of the absorbent article also contributes to hinder the growth of microorganisms, which are known to be responsible for the generation of malodorous compounds. For instance the breathability increases the oxygen quantity inside the absorbent article which results in the decrease of the number of anaerobic microorganisms which are known to be responsible for the generation of malodorous compounds. Without to be bounded by theory, it is speculated that this change in the anaerobic-aerobic microorganism equilibrium associated to the presence of an increased level of oxygen results in the full use of the oxidizing capacity of the oxidizing agent against aerobic microorganisms (which are also known to be responsible for the generation of malodorous compounds) which would otherwise, in a more dominant anaerobic environment have dominantly oxidized the anaerobic microorganisms. Thus the amount of odours associated with the presence of microorganisms is reduced by the absorbent articles of the present invention.

Furthermore, the oxidizing agent itself is able to prevent the generation of odour by blocking enzymatic and/or microbial activity. Indeed it is speculated that the oxidising agents herein (preferably the peroxyacids as described herein and/or the diacyl peroxides as described herein) oxidise sensitive sulphidryl and sulphur bonds typically present in enzymes, thereby deactivating the enzymes which otherwise would have contributed to the normal metabolism of the micro-organisms. It is further speculated that these oxidising agents oxidise double bonds in other metabolites like for instance nutriments (e.g., unsaturated fat) for the micro-organisms, thereby rendering these nutriments inefficient for the microbial growth which otherwise would have resulted in generation of malodorous compounds. It is further believed that the oxidising agents described herein disrupts the chemiosmotic function of the lipoprotein cytoplasmatic membrane of the microbe/bacteria cells and thus disrupt the transport function at the cell walls. This later disruption is especially noticeable with the hydrophobic oxidising agents like the cyclic or cyclic alkyl peroxyacids namely the ones according to the chemical formulae described herein after and/or the diacyl peroxides described herein after. By 'hydrophobic oxidising agent' it is meant those oxidising agents that can be solubilised in the lipidic layers of the cell wall of the micro-organisms. Highly preferred hydrophobic oxidising agents for use herein are the pthalimido- and phtalamido peroxyalkanoic acids, dibenzoyl peroxide, benzoyl lauroyl peroxide and/or dilauroyl peroxide.

The present invention also uses odour control agents having the advantage not only of preventing the formation of malodours odour for example by the mechanism described herein before but also to combat the malodorous compounds already present by oxidising them into non-smelling compounds.

Advantageously, the breathability of the absorbent article further results in increased movement of the volatile malodorous compounds. Hence, the amount of actual physical contact between these compounds and the oxidising odour control agents increases. Contact between the oxidizing odour control agents and the malodorous compounds is usually required in order to effectively combat the odorous compounds. This increased amount of contact between the malodorous compounds and the oxidising agents further contributes to significantly improve the effectiveness of the oxidising odour control agents. Hence this further contributes to use less oxidizing agent within the absorbent article whilst maintaining the required level of odour control.

Advantageously, the reduction in the hot, humid and occlusive environment between the vicinity of the skin of the wearer and the wearer facing surface of the absorbent article itself also reduces the tendency of the wearer of the product to perspire. Consequently, the amount of associated perspiration related odour will be reduced. Thus, the breathability of the article actually reduces the amount of odour generated within the absorbent article. As a result the odour control system works more effectively on the remaining odorous compounds present in the article.

A further important advantage associated with the absorbent articles of the present invention is that they deliver a better feeling and more acceptable cleanness level for the person wearing them. Indeed, the presence of the oxidising agent according to the present invention provides absorbent articles with controlled odour capacity (including odour prevention and odour destruction by oxidation) when the menstruation comes into contact with the article, but also absorbent articles being capable of changing the color of the menstruation (red blood color) to a pale red color and even to a whitish color.

Yet an additional benefit of the present invention is that the combination of breathability and oxidising agent leads to an improvement in the overall dryness of the product. The breathability of the article allows for the evaporation of fluid from the article, and also as indicated above a reduction in the amount of perspiration generated by the wearer of the product and thus a reduction in hot and sweaty feelings often associated with the presence of topsheets designed to retain a clean and dry surface. The article therefore needs to retain less fluid and can do so more effectively. Furthermore the oxidizing odour control systems are also typically able to disrupt the fluid thereby increasing its evaporation and thus improves the dryness of the product.

### Background art of the invention

The incorporation of breathable backsheets in absorbent articles for improved wearer comfort has been described in the art such as for example in GB 2 184 389, US 3 881 489 and EPO 203 821. However, none of these prior art documents recognize the benefits of the combination of a breathable absorbent article by the utilization of a breathable backsheet, with an oxidising agent-containing odour control system.

EP-A-811 392 discloses breathable absorbent articles having a chelating agent based odor control system. However, no oxidising agents are disclosed.

### Summary of the Invention

The present invention relates to an absorbent article, having a breathable backsheet and further comprising an odour control system comprising an oxidising agent. The combination of the oxidising agent-containing odour control system and the breathability of the absorbent article provides an unexpected improvement of the odour control performance of the oxidizing agent.

### Detailed Description of the Invention

The present invention relates to breathable absorbent articles such as sanitary napkins, panty liners, incontinence devices and baby diapers, interlabial pads. Typically such products comprise a liquid pervious topsheet, a backsheet and an absorbent core intermediate the topsheet and the backsheet. According to the present invention the breathability of the sanitary napkin is provided by the presence of a breathable backsheet which thereby allows the circulation of water vapour and preferably both water vapour and air through it. According to the present invention the absorbent article further comprises an odour control system comprising an oxidising agent. It has now been found that the combination of the breathability of the absorbent article and the odour control system comprising an oxidising agent results in an unexpected improvement of the performance of the oxidising agent-containing odour control system.

By "bodily fluids" it is meant herein any fluids produced by human body including for instance perspiration, urine, menstrual fluids, faeces, vaginal secretions and the like.

### Odour control system

According to the present invention the odour control system comprises as an essential component an oxidising agent.

The oxidising agents according to the present invention can be any oxidising agent known to those skilled in the art able to reduce and prevent malodour . They include oxygen bleaches and/or hypohalite bleaches.

Hypohalite bleaches may be provided by a variety of sources, including bleaches that are oxidative bleaches and subsequently lead to the formation of positive halide ions as well as bleaches that are organic based sources of halides such as chloroisocyanurates.

Suitable hypohalite bleaches for use herein include the alkali metal and alkaline earth metal hypochlorites, hypobromites, hypoiodites, dichloroisocyanurates (e.g., potassium and sodium dichloroisocyanurates), trichlorocyanurates (e.g., potassium and sodium trichlorocyanurates), N-chloroimides, N-chloroamides, N-chloroamines, chlorohydantoins, chlorinated trisodium phosphate dodecahydrates, chloramine T, chlorine dioxide or mixtures thereof.

The preferred hypohalite bleaches among the above described are the alkali metal and/or alkaline earth metal hypochlorites such as lithium hypochlorite, calcium hypochlorite, potassium hypochlorite, magnesium hypochlorite, as well as other hypohalite bleaches like chlorinated trisodium phosphate dodecahydrate, potassium dichloroisocyanurate, sodium dichloroisocyanurate, potassium trichlorocyanurate, sodium trichlorocyanurate, other alkali metal salts or mixtures thereof.

Suitable oxygen bleaches for use herein include peroxygen bleaches or mixtures thereof.

Such peroxygen bleaches include hydrogen peroxide, percarbonates, persilicates, persulphates, perphosphates, perborates, peroxyacids, alkyl hydroperoxides, peroxides, diacyl peroxides, ozonides, supereoxides, oxo-ozonides, periodates, salts thereof or mixtures thereof. Peroxyacids and diacyl peroxides are preferred herein.

Suitable hydroperoxides for use herein include tert-butyl hydroperoxide, cumyl hydroperoxide, 2,4,4-trimethylpentyl-2-hydroperoxide, di-isopropylbenzene-monohydroperoxide, tert-amyl hydroperoxide and 2,5-dimethyl-hexane-2,5-dihydroperoxide.

Suitable peroxides for use herein include for example lithium peroxide, sodium peroxide, potassium peroxide, ammonium peroxide, calcium peroxide, rubidium peroxide, cesium peroxide, stromtium peroxide, barium peroxide, magnesium peroxide, mercury peroxide, silver peroxide, zirconium peroxide, hafnium peroxide, titanium peroxide, phosphorus peroxide, sulphur peroxide, rhenium peroxide, iron peroxide, cobalt peroxide, nickel peroxide, other alkali metal salts thereof or alkaline earth metal salts thereof or mixture thereof.

Suitable superoxides for use herein include for example lithium superoxide, sodium superoxide, potassium superoxide, calcium superoxide, rubidium superoxide, cesium superoxide, strontium superoxide, barium superoxide, other alkali metal salts thereof or alkaline earth metal salts thereof or mixture thereof.

Suitable ozonides for use herein include for example lithium ozonide, sodium ozonide, potassium ozonide, rubidium ozonide, cesium ozonide, ammonium ozonide, tetramethyl ammonium ozonide, stromtium ozonide, barium ozonide, magnesium ozonide, other alkali metal salts thereof or alkaline earth metal salts thereof or mixture thereof.

Suitable perphosphates for use herein include for example lithium perphosphate, sodium perphosphate, potassium perphosphate, zinc perphosphate, ammonium perphosphate, calcium perphosphate, barium perphosphate, magnesium perphosphate, other alkali metal salts thereof or alkaline earth metal salts thereof or mixture thereof.

Suitable perborates for use herein include for example sodium perborate, potassium perborate, ammonium perborate or other alkali metal salts thereof or alkaline earth metal salts thereof or mixture thereof.

Suitable persulphates for use herein include sodium persulphate, potassiumdipersulphate, potassium persulphate as well as other alkali metal salts thereof or mixture thereof.

Other suitable peroxygen bleaches also include dicetylperoxydicarbonate, 1,1bis(terbutylperoxy)-3,3,5-trimetylcyclohexane, di(1-naphtyl) peroxide, tert-butyl perbenzoate, O,O,-t-buthyl-O-isopropyl mono-peroxycarbonate, percarbonates like stearyl percarbonate, 2-ethylhexyl percarbonate and sec-butyl percarbonate and corresponding perborates and persulphates.

Suitable diacyl peroxides for use herein are according to the formula :

R1-C(O)-O-O-(O)C-R2

wherein R1 and R2 can be the same or different and are selected from the group of substituted or unsubstituted, saturated or unsaturated, linear, branched or cyclic hydrocarbon groups having from 1 to 50 carbon atoms, preferably from 2 to 40 and more preferably from 4 to 30 carbon atoms.

Suitable diacyl peroxides for use herein include those wherein R1 and R2 are independently an aliphatic group, those wherein R1 and R2 are independently an aromatic ring and those wherein R1 is an aliphatic group and R2 is an aromatic ring.

Typically R1 and R2 are independently an aliphatic group having from 2 to 40, more preferably 4 to 30, even more preferably 5 to 20 carbon atoms. These aliphatic groups may be linear, branched, cyclic, saturated, unsaturated, substituted, unsubstituted or mixtures thereof. Preferably the aliphatic groups are linear and comprise from 4 to 20 carbon atoms, and more preferably 8 to 18 carbon atoms. Where such an aliphatic group is substituted, the carbon atom is preferably substituted with a halide or sulfate-containing or nitrogen-containing functionality such as SO3-, SO4-, NO2, NR3+ where R = H or an alkyl chain containing from 1 to 5 carbon atoms.

Typically R1 and R2 may be independently a mono or polycyclic aromatic ring, a homo or heteroaromatic ring, substituted or unsubstituted having from 2 to 50 carbon atoms and mixtures thereof. Where such an aromatic ring is substituted, the carbon atom is preferably substituted with a halide, sulphur-containing group, nitrogen-containing group or an alkyl chain wherein the number of carbon atoms ranges from 1 to 20, most preferably from 4 to 10. Suitable sulphur-containing or nitrogen-containing substituents include SO3-, SO4-, NO2, NR3+ where R=H or an alkyl chain containing from 1 to 5 carbon atoms. Preferred aromatic ring is benzene.

Particularly suitable diacyl peroxides for use herein are those wherein R1 is an aliphatic group as defined herein before and R2 is a mono or polycyclic aromatic ring, a homo or heteroaromatic ring, substituted or unsubstituted as defined herein before. Such preferred diacyl peroxides are benzoyl alkanoyl peroxides wherein the alkanoyl group has from 4 to 20 carbon atoms and more preferably from 8 to 18 carbon atoms.

Suitable diacyl peroxides for use herein are dilauroyl peroxide, didecanoyl peroxide, dimyristoyl peroxide, dibenzoyl peroxide, di-4-methylbenzoyl peroxide, di-p-methoxy-benzoyl peroxide, acetyl benzoyl peroxide, benzoyl stearoyl peroxide, benzoyl decanoyl peroxide, benzoyl cetyl peroxide, para-alkyl benzoyl lauroyl peroxide, para-alkyl benzoyl decanoyl peroxide, para-alkyl benzoyl cetoyl peroxide, di-4-phenylbenzoyl peroxide, di-t-butylperoxide, t-butyl cumyl peroxide, diethyl peroxide, diacetyl peroxide, dicumyl peroxide, diheptanoyl peroxide, didecanoyl peroxide, benzoyl lauroyl peroxide, diheptanoyl peroxide, distearoyl peroxide, disuccinyl peroxide, 3,5,5-trimethylhexanoyl peroxide, or mixtures thereof.

Highly preferred diacyl peroxides herein are dilauroyl peroxide which may be commercially available as flakes from AKZO NOBEL under the name Laurox®, or as powder under the name Laurox S®, or in suspension in water under the name Laurox W 40®, or dibenzoyl peroxide which may be commercially available from AKZO NOBEL under the name Lucidol® in powder form or Lucidol W40® in the form of a suspension in water, and/or benzoyl lauroyl peroxide.

The aromatic alkanoyl peroxides described herein like benzoyl lauroyl peroxide are easily synthesized by persons skilled in the art, see for example Organic Peroxides Vol. 1; page 65, edited by Daniel Swern of Wiley Interscience.

Suitable peroxyacids for use herein are according to the following formula:

R₃-CO3H

wherein R₃ is a substituted or unsubstituted, saturated or unsaturated, linear or branched hydrocarbon group having from 1 to 25 carbon atoms, or a cyclic group having from 3 to 32 carbon atoms and optionally at least one heteroatom, or a cyclic alkyl group having from 4 to 32 carbon atoms and optionally at least one heteroatom.

Typically R₃ is a substituted or unsubstituted, linear or branched alkyl group or alkenyl group having from 1 to 25 carbon atoms, more preferably from 1 to 14 carbon atoms, even more preferably from 3 to 10, and most preferably from 4 to 6. R₃ may also typically be an aryl group having from 3 to 32 carbon atoms, preferably from 3 to 25, more preferably from 6 to 20, even more preferably from 8 to 15 carbons atoms, or an aryl alkyl group having from 4 to 32 total carbon atoms, preferably from 4 to 25, more preferably from 6 to 20 and even more preferably from 8 to 13, or an heterocyclic group containing from 3 to 32 carbon atoms, preferably from 3 to 25, more preferably from 3 to 20 carbon atoms, even more preferably from 5 to 15 and from 1 to 5 hetero atoms, preferably 1 to 3, wherein the hetero atoms are independently selected from the group consisting of oxygen, nitrogen and sulfur, and preferably is nitrogen or oxygen, or an heterocyclic alkyl group containing from 4 to 32 total carbon atoms, preferably from 4 to 25, more preferably from 4 to 22, even more preferably from 6 to 18 and from 1 to 5 hetero atoms, preferably 1 to 3, wherein the hetero atoms are independently selected from the group consisting of oxygen, nitrogen and sulfur, and preferably is nitrogen or oxygen.

The preferred peroxyacids according to the present invention are those wherein R₃ is a cyclic group or cyclic alkyl group, preferably a heterocyclic group or heterocyclic alkyl group.

Even more preferred herein are the peroxyacids according to the following formulae: 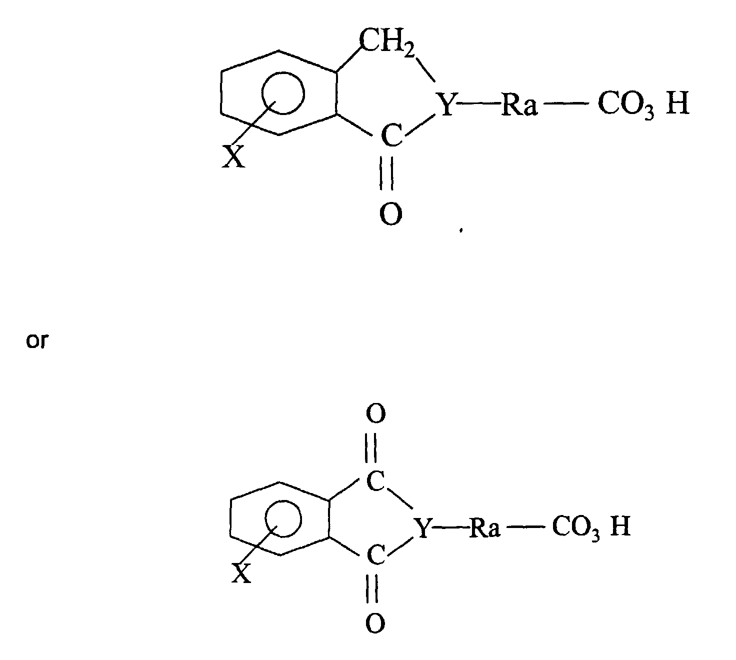 wherein Ra is a substituted or unsubstituted, saturated or unsaturated, linear or branched hydrocarbon group having from 1 to 14 carbon atoms, Y is an heteroatom and X are substituents in position ortho or meta independently selected from the group of hydrogen, hydroxy, halogen, carboxy, aliphatic saturated or unsaturated, linear or branched, hydrocarbon group having from 1 to 10 carbon atoms.

Preferably Ra is a substituted or unsubstituted, linear or branched alkyl group or alkenyl group having from 2 to 12 carbon atoms, preferably from 2 to 10, more preferably from 2 to 8, even more preferably from 3 to 6 and most preferably 5 carbon atoms. Preferably Y is an heteroatom selected from the group consisting of oxygen, nitrogen and sulfur, and more preferably is nitrogen atom (>N-). Preferably X are substituents on position ortho or meta independently selected from the group consisting of hydrogen, hydroxy, aliphatic linear or branched alkyl group or alkenyl group having from 1 to 10 carbon atoms, preferably from 2 to 7 and most preferably from 3 to 5 carbon atoms. Highly preferred herein all the substituents X are independently hydrogen.

The preferred peroxyacids for use according to the present invention are pthalimido- and phtalamido peroxyalkanoic acids.

Highly preferred herein is ε-pthalimido peroxyhexanoic acid which may be commercially available from AUSIMONT under the name PAP ®, or EURECO ® (in granule form), Eureco WKC ® (in wet granule form) or Eureco HC ® (in powdered active form).

Other suitable oxidising agents for use, herein include inorganic oxidants like urea peroxide, potassium permanganate, potassium chromate, potassium dichromate, ruthenium tetra-oxide, osmium tetra-oxide and the like, cerium compounds including cerium oxides, cerium hydroxides, cerium hydrated oxides, cerium oxysalts and the like, lead compounds including lead oxides, lead tetraoxides, lead acetates, lead tetracetates and the like, manganese compounds including manganese permanganate, manganese oxide and the like, metallic salts like silver, copper, zinc, ferric and cupric salts (e.g., silver nitrate, silver nitrite, ferric chloride, cupric chloride, cupric acetate), quinones and derivatives, ozone, hydrazine and derivatives thereof and the like.

According to the present invention the absorbent articles typically comprise from 0.5 g/m² to 300 g/m², preferably from 3g/m² to 250 g/m², more preferably from 6g/m² to 150 g/m² of said oxidising agent or a mixture thereof.

### Optional odour control agents

According to the present invention the odour control system may comprise in addition to said oxidising agent at least one additional odour control agent known in the art.

For instance additional odour control agent or combinations thereof, known in the art for this purpose may be used herein. These agents can typically be classified according to the type of odour the agent is intended to combat. Odors may be chemically classified as being acidic, basic or neutral.

Alternatively, the odor control agents may be categorised with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carboxylic acids such as citric acid, lauric acid, boric acid, adipic acid and maleic acid, activated carbons, clays, zeolites, silicas, absorbent gelling materials (AGM), masking agents, antimicrobials, chitin, kieselguhr, carbonates (e.g., sodium carbonate), bicarbonates (e.g., sodium bicarbonate), phosphates (e.g., sodium phosphate), sulphates (e.g., zinc and copper sulphates) and starches. Such odor control agents and systems are disclosed in more details hereinafter and for example in EP-A- 348 978, EP-A- 510 619, WO 91/12029, WO 91/11977, WO 91/12030, WO 81/01643 and WO 96/06589. Also cyclodextrin and derivatives thereof may be used as described in US 5429628.

Other suitable odour control agents for use herein also include chelating agents and may be selected from amino carboxylates such as for example ethylenediamine- tetracetate, as described for example in US 4356190, amino phosphonates such as ethylenediaminetetrakis (methylene- phosphonates), polyfunctionally-substituted aromatic chelating agents as described in US 3 812 044 and mixtures thereof. Without intending to be bound by theory it is believed that the benefit of these materials is in part due to their exceptional ability to remove iron, copper, calcium, magnesium and manganese ions present in the absorbed fluids and their degradation products by the formation of chelates.

Another suitable odor control agent for use herein is a buffer system, such as citric acid and sodium bicarbonate, sodium phosphate and sorbic acid buffer systems. Also, buffer systems having a pH of from 7 to 10 as described for example in WO94/25077 may be useful herein.

Alternative odor control agents are ion exchange resins such as those described in US 4 289 513 and US 3340875.

Masking agents such as perfumes may also be used as odor control agents herein.

The amount of the optional additional odour control agent or mixture thereof used in absorbent article will typically range from 0.5 g/m² to 300 g/m², preferably from 3g/m² to 250 g/m², more preferably from 6g/m² to 150 g/m².

### Absorbent gelling odor control materials

As is well-known from recent commercial practice, absorbent gelling materials (sometimes referred to as "super-sorbers") are becoming broadly used in absorbent articles. AGM's are materials which have fluid-absorbing properties.

Such materials are highly preferred herein as the optional additional odor control agent due to their dual function of absorbing fluids and odors.

Such materials form hydrogels on contact with water (e.g., with urine, blood, and the like). One highly preferred type of hydrogel-forming, absorbent gelling material is based on polyacids, especially polyacrylic acid. Hydrogel- forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked,. hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 10 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent article, which is undesirable from a consumer aesthetics standpoint. Furthermore, rate of fluid absorption can be affected by particle size. Larger particles have very much reduced rates of absorption. Preferred for use herein are absorbent gelling material s particles substantially all of which have a particle size of from about 30 microns to about 2mm. "Particle Size" as used herein means the weighted average of the smallest dimension of the individual particles.

The amount of absorbent gelling material particles used in absorbent article will typically range from 10g/m² to 150g/m², preferably from 30g/m² to 150g/m², more preferably from 55g/m² to 100 g/m².

### Silica odor control agent

Particularly suitable herein as an additional odor control agent is silica. Silica, i.e. silicon dioxide SiO₂ exists in a variety of crystalline forms and amorphous modifications, any of which are suitable for use herein. In particular, silicas having a high surface area or in agglomerated form are preferred. Silica molecular sieves are not considered to be within the definition of silica as used herein. Preferably the silica is in a highly purified form such that is contains at least 90%, preferably 95%, more preferably 99% silicon dioxide. Most preferably the silica is silica gel having a 100% silica content. Alternatively, the silica may be provided from other sources such as metal silicates including sodium silicate.

### Zeolite odor control agent

The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8.

Zeolites are crystalline aluminosilicates of group IA and group IIA elements such as Na, K, Mn, Ca and are chemically represented by the empirical formula :

M_{2/n}O . Al₂O₃. ySiO₂ . wH₂O

where y is 2 or greater, n is the cation valence, and w is the water content in the voids of the zeolite.

Structurally, zeolites are complex, crystalline inorganic polymers based on an infinitely extending framework of AlO₄ and SiO₄ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

M_{x/n} [(AlO₂)ₓ (SiO₂)_{y} ] . WH₂O

where n is the valence of cation M, w is the number of water molecules per unit cell, x and y are the total number of tedrahedra per unit cell, y/x usually having values of 1-5.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred is zeolite A.

According to the present invention the zeolite is preferably hydrophobic. This is typically achieved by increasing the molar ratio of the SiO₂ to AlO₂ content such that the ratio of x to y is at least 1, preferably from 1 to 500, most preferably from 1 to 6.

Preferred additional odour control agents are zeolites, silica, activated carbon, AGM, or mixtures thereof. Preferred odour control systems for use herein include the following combinations such as oxidising agent and absorbing gelling materials and the combination of oxidising agent, zeolites and absorbing gelling materials and also the combination of oxidising agent, zeolites, silica and absorbent gelling materials. An advantage of the combination of the oxidising agent and additional odour control agents, in particular the absorbing materials such as AGM in the breathable absorbent article is the promotion of improved dryness of the absorbent article.

In a preferred embodiment herein the ratio of said oxidising agent to said additional odour control agents is from 1:10 to 10:1, preferably from 1:5 to 5:1, more preferably from 1:3 to 3:1.

### The absorbent article

The oxidising agent-containing odour control system of the present invention may be incorporated into the absorbent article by any of the methods disclosed in the art, for example, the system may be layered on the core of the absorbent material or mixed within the fibres of the absorbent core. The odour control system is preferably incorporated between two layers of cellulose tissue. Optionally the odour control system may be bonded between two cellulose tissue layers with for example a hot melt adhesive (e.g., polyethylene powder) or any suitable bonding system like for instance glue commercially available from ATO Findley under the name H20-31® in absence of heating.

The oxidising agent and additional optional odour control agents may be incorporated as a powder or a granulate or can be sprayed in the from of an oxidising agent-containing solution within the absorbent article.

The oxidizing agent may be distributed homogeneously or non-homogeneously over the entire absorbent article or in at least one layer of the topsheet or in at least one layer of the core or any mixtures thereof. The oxidising agent may be distributed homogeneously or non-homogeneously on the whole surface of the desired layer or layers, or on one or several area of the surface layer/layers to which it is positioned (e.g., central area and/or surrounding area like the edges of a layer of the absorbent article) or a mixture thereof.

If additional odour control agents are present, the oxidising agent is positioned such that at least a portion of the fluid discharge comes into contact with the oxidising agent before the optional odour control agent. More preferably, the oxidising agent is located towards or within the topsheet itself and the additional optional odour control agents are located further away from the topsheet than the oxidising agent, preferably towards the backsheet. In a preferred embodiment the oxidising agent is located in a separate layer from the optional odour control agents. Most preferably the oxidising agent is positioned within at least one of the topsheet layers and the additional odour control agent is positioned within the core.

### Backsheet

According to the present invention, the absorbent articles comprise as an essential component a breathable backsheet. The primary role of the breathable backsheet is to prevent the extrudes absorbed and contained in the absorbent article from welling articles that contact the absorbent article such as pyjamas and undergarments. In order to achieve this the backsheet typically extends across the whole of the absorbent structure and may extend into and form part of or all of sideflaps, side wrapping elements or wings. In addition to the prevention of liquid transport through the backsheet however, the breathable backsheet also permits the transfer of water vapour and preferably both water vapour and air through it and thus allows the circulation of air into and out of the backsheet and the absorbent article itself.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

Particularly preferred are backsheets meeting the requirements as defined in European Patent Application number 96830343.8 and more preferably wherein the absorbent article also meets the requirements as described therein.

According to the present invention the breathable backsheet comprises at least one, preferably at least two water vapour permeable layers. Suitable water vapour permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 637 819 and US 4 591 523.

Suitable macroscopically expanded films for use herein include films as described in for example US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours International S.A., Switzerland.

According to the present invention the backsheet may comprise in addition to said water vapour permeable layer additional backsheet layers. Said additional layers may be located on either side of said water vapour permeable layer of the backsheet. The additional layers may be of any material, such as fibrous layers or additional water vapour permeable layers as described herein above.

### Absorbent core

According to the present invention the absorbent articles may further comprise a topsheet and absorbent core. The absorbent material or core can be a fluffy fibrous absorbent core, comprising hydrogel particles if desired, or laminated tissues with or without particulate materials including hydrogel particles. The absorbent core fibres can be any of those known in the art including cellulose fibres or polymeric fibres rendered absorbent or even non absorbent matrix fibres. Also tissues of sufficient basis weight and absorbency can be used in the absorbent core according to the present invention.

### Topsheet

According to the present invention the topsheet may comprise a single layer or a multiplicity of layers. In a preferred embodiment the topsheet comprises a first layer which provides the user facing surface of the topsheet and a second layer between the first layer and the absorbent structure/core. The topsheet provides a layer through which the liquids to be absorbed penetrate to the absorbent material.

The topsheet as a whole and hence each layer individually needs to be compliant, soft feeling, and non-irritating to the wearers skin. It also can have elastic characteristics allowing it to be stretched in one or two directions. Typically, the topsheet extends across the whole of the absorbent structure and can extend into and form part of or all of the preferred sideflaps, side wrapping elements or wings. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523.

According to the present invention the absorbent article is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof. Preferably the breathable backsheet is bonded to other elements of the absorbent article so as to minimise and preferably eliminate any reduction in the vapour permeability of the backsheet.

According to the present invention the absorbent article may find utility as sanitary napkins, panty liners, adult incontinence products and baby diapers. The present invention finds particular susceptibility as sanitary napkins and panty liners. Thus in addition to the components described herein above, the absorbent article may also comprise all those features and parts which are typical for products in the context of their intended use such as wings and side flaps, undergarment adhesive means, release paper, wrapping elements, fastening means and the like.

The present invention is further illustrated by the following examples

### Examples

### Example 1:

This is an example of a panty liner according to the present invention and is a modified panty liner based on Always "Alldays Duo Active" manufactured by Procter & Gamble, Germany. The topsheet is a film/non woven composite {film supplier code BPC 5105 CPM BP Chemical Germany, non woven supplier code ARBO TB/BI Mequinenza Spain}. The core material is a tissue laminate (13.2 cm x 4.0 cm) composed of a 2 layers of airlayed tissue of 55 g/m² basis weight {available from Unikay Italy under the supplier code Unikay 303 LF}. Between the two tissue layers the laminate contains as the odour control system an oxidizing agent {ε-pthalimido peroxyhexanoic acid available from Ausimont under the name PAP®} at a basis weight of 30 g/m².

The backsheet comprises two layers a first layer and a second layer. The first layer is in contact with the absorbent tissue and the second layer. The second layer is in contact with the first layer and the undergarment of the wearer. The first layer is a formed apertured film (CPT) made of Low Density PE {supplied by Tredegar Film Products B.V. Holland under the manufacturing code X-1522}. The second layer is composed of a nonwoven laminate {14MB/14SB manufactured by Corovin GmbH in Germany under the trade name MD 2005}. The nonwoven laminate is composed of 14 g/m² spunbond and 14 g/m² meltblown. Each backsheet layer is joined over the full surface by a extensively overlapped spiral glue application at a basis weight of approximately 8 g/m². The glue utilised for attachment of both backsheet layers was supplied by SAVARE' SpA. Italy (under the material code PM17).

### Example 2:

Example 2 is identical to example 1 except that the second layer of the backsheet has been replaced by a nonwoven laminate composed of 16g/m² spunbond and 6 g/m² meltblown {supplied under the code of SM 22-6PH by Union SpA, ltaly}.

### Example 3:

Example 3 is identical to example 1 except that the odour control system further comprises AGM (available from DOW Chemicals Germany under the supplier code; DOW XZ 95890.1) at a basis weight of 60 g/m^{2.}

### Example 4:

Example 4 is identical to example 3 except that the second layer of the backsheet has been replaced by a nonwoven laminate composed of 16g/m² spunbond and 6 g/m² meltblown {supplied under the code of SM 22-6PH by Union SpA, Italy}.

### Example 5:

This is an example of a sanitary napkin according to the present invention. The sanitary napkin is based on an Always Ultra sanitary napkin available from Procter & Gamble Germany which has been modified. The topsheet is a CPM material available from Tredegar Film Products B. V. Holland under the code X-1522. The core material is a tissue laminate (20.7 cm x 7.0 cm) composed of a 2 layers of airlayed tissue of 55 g/m² basis weight (available from Unikay Italy under the supplier code Unikay 303 LF}. Between the two tissue layers the laminate contains an odour control system of AGM (available from DOW Chemicals Germany under the supplier code; DOW XZ 95890.1) at a basis weight of 60 g/m², a zeolite (available from Degussa Germany under the supplier code; Wessalith CS) at a basis weight of 61 g/m² and ε-pthalimido peroxyhexanoic acid (available from Ausimont under the supplier name PAP®) at a basis weight of 35g/m². The core laminate was manufactured and supplied by Korma Italy (under the experimental manufacturing code: XA 070.01.003). The sanitary napkin has a multi-layer breathable backsheet comprising a formed apertured film backsheet layer and a second nonwoven layer. The first layer is a blend of low and high density PE with a crush resistant hexagonal hole configuration {supplied by Tredegar Film Products B.V. Holland under the manufacturing code AS 225 HD 25}. The second layer is an improved nonwoven laminate composed of 3 layers with basis weights 14g/m² spunbond - 20 g/m² meltblown - 14 g/m² spunbond (manufactured by Corovin GmbH in Germany under the trade name MD 3005).

### Example 6:

Example 6 is identical to example 5 except that the second layer of backsheet has been replaced by a microporous layer ( manufactured by Exxon Chemical Company in Illinois under the name Exxon XBF 112W) composed of Low Density PE and calcium carbonate particles at basis weight of 35 g/m2.

## Claims

1. An absorbent article comprising a liquid permeable topsheet, a breathable backsheet and an absorbent core, said core being intermediate said topsheet and said backsheet, said absorbent article comprising an odour control system comprising an oxidising agent.

2. An absorbent article according to claim 1, wherein said oxidising agent is selected from the group consisting of peroxygen bleaches, hypohalite bleaches, inorganic oxidants, cerium compounds, lead compounds, manganese compounds, metallic salts, quinones and derivatives, ozone, hydrazine and derivatives thereof, and mixture thereof, preferably is hydrogen peroxide, a percarbonate, a persilicate, a persulphate, a perphosphate, a perborate, a peroxyacid, an alkyl hydroperoxide, a peroxide, a diacyl peroxide, an ozonide, a supereoxide, an oxo-ozonide, a periodate, a salt thereof or a mixture thereof and more preferably is a peroxyacid and/or a diacyl peroxide.

3. An article according to any of the preceding claims wherein said oxidizing agent is:
- a peroxyacid according to the following formula:
R₃-CO3H
wherein R₃ is a substituted or unsubstituted, saturated or unsaturated, linear or branched hydrocarbon group having from 1 to 25 carbon atoms or a cyclic group having from 3 to 32 carbon atoms and optionally at least one heteroatom, or a cyclic alkyl group having from 4 to 32 carbon atoms and optionally at least one heteroatom,
- or a diacyl peroxide according to the formula :
R1-C(O)-O-O-(O)C-R2
wherein R1 and R2 are the same or different and are selected from the group of substituted or unsubstituted, saturated or unsaturated, linear, branched or cyclic hydrocarbon groups having from 1 to 50 carbon atoms, preferably from 2 to 40 and more preferably from 4 to 30 carbon atoms, or a mixture thereof.

4. An article according to claim 3 wherein R1 and R2 in the diacyl peroxide formula are independently a mono or polycyclic aromatic ring, a homo or heteroaromatic ring, substituted or unsubstituted of from 2 to 50 total carbon atoms, preferably a benzene, or an aliphatic group having from 2 to 40 carbon atoms and wherein the diacyl peroxide is most preferably a benzoyl alkanoyl peroxide wherein the alkanoyl group has from 4 to 20 carbon atoms or a mixture thereof

5. An article according to any of the preceding claims wherein the oxidising agent is a peroxyacid according to the formulae: 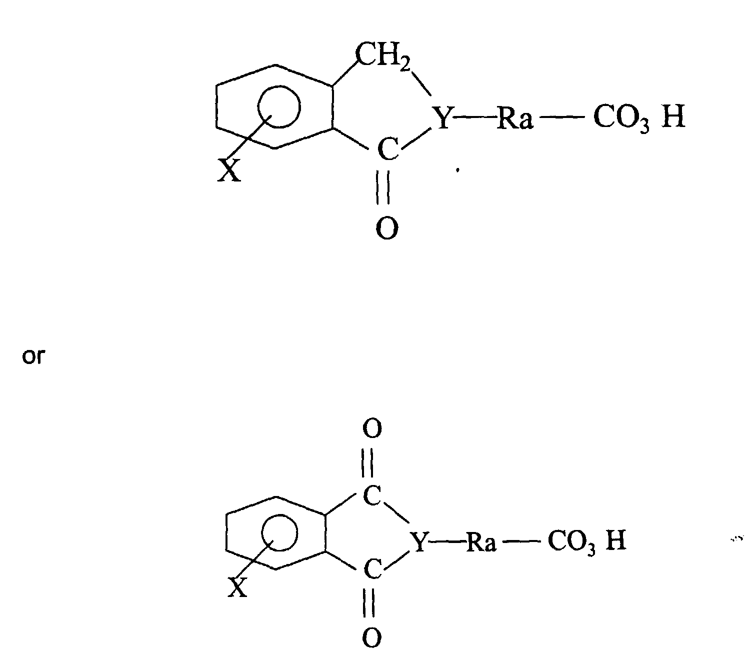 wherein Ra is a substituted or unsubstituted, saturated or unsaturated, linear or branched hydrocarbon group having from 1 to 14 carbon atoms, Y is an heteroatom, preferably selected from the group consisting of oxygen, nitrogen and sulfur and more preferably is nitrogen, and X are substituents in position ortho or meta independently selected from the group of hydrogen, hydroxy, halogen, carboxy, aliphatic saturated or unsaturated, linear or branched, hydrocarbon group having from 1 to 10 carbon atoms, or a mixture thereof.

6. An article according to any of the preceding claims wherein the oxidizing agent is a pthalimido peroxyalkanoic acid, a phtalamido peroxyalkanoic acid, dilauroyl peroxide, dibenzoyl peroxide and/or benzoyl lauroyl peroxide.

7. An article according to any of the preceding claims which comprises from 0.5 g/m² to 300 g/m², preferably from 3g/m² to 250 g/m², more preferably from 6g/m² to 150 g/m² of said oxidizing agent or a mixture thereof.

8. An absorbent article according to any one of the preceding claims, wherein said odour control system further comprises at least one additional odour control agent.

9. An absorbent article according to claim 8, wherein said odour control system further comprises as the additional odour control agent, absorbing gelling materials, silicas, zeolites, carbons, starches, cyclodextrine, chelating agents, pH buffered materials, chitin, kieselguhr, clays, ion exchange resins, carbonates, bicarbonates, phosphates, sulphates, masking agents or combination thereof and preferably is absorbing gelling agent, silicate, zeolite or a combination thereof.

10. An article according to any of the preceding claims 8 or 9 which comprises from 0.5 g/m² to 300 g/m², preferably from 3g/m² to 250 g/m², more preferably from 6g/m² to 150 g/m² of said additional odour control agent or a mixture thereof.

11. An absorbent article according to any one of the preceding claims, wherein said breathable backsheet comprises at least one layer selected from an apertured polymeric film or a 2-dimensional planar apertured film.

12. An absorbent article according to claim 11, wherein said layer is a 2 dimensional planar apertured layer, wherein said apertures have an average diameter of from 150 micrometers to 5 micrometers.

13. An absorbent article according to claim 11, wherein said layer is an apertured polymeric film, wherein said apertures have an average diameter of from 100 micrometers to 500 micrometers.

14. An absorbent article according to claim 11, wherein said breathable backsheet comprises at least two layers, a first layer comprising an apertured layer and a second layer comprising a fibrous layer.

15. An absorbent article according to any one of the preceding claims, wherein said article is a sanitary napkin or a panty liner.
